# EUROPEAN PATENT APPLICATION

(11) **EP 3 338 758 A1**
(43) Date of publication of application: **27.06.2018**
(21) Application number: 16838783.5
(22) Date of filing: 12.08.2016
(51) Int. Cl.: A61K 8/19, A61K 8/22, A61K 8/46, A61Q 5/04

(54) **METHOD FOR SHAPING HAIR BY CURLY HAIR-CORRECTION TREATMENT OR PERMANENT WAVING TREATMENT, AND HAIR TREATMENT SOLUTION FOR SHAPING HAIR**

(30) Priority: 21.08.2015 JP 2015164287; 24.08.2015 JP 2015165004; 21.04.2016 JP 2016084919
(71) Applicant: ICTB Global Co., Ltd., Yokohama-shi, Kanagawa 221-0825 (JP)
(72) Inventor: SHIODA, Masataka, Yokohama-shi Kanagawa 221-0825 (JP); SHIODA, Keita, Yokohama-shi Kanagawa 221-0825 (JP)
(74) Representative: Vossius & Partner Patentanwälte Rechtsanwälte mbB
(86) International application number: PCT/JP2016/003721
(87) International publication number: WO 2017/033432

(57) **Abstract**

Disclosed is a method for hair shaping treatment that is straightening treatment or permanent waving treatment, including at least the steps of: applying a first agent containing a reducing agent to hair, and leaving the hair for a predetermined period of time; washing off the first agent applied to the hair; applying a second agent containing an oxidizing agent to the hair, and leaving the hair for a predetermined period of time; and washing off the second agent, wherein the method for hair shaping treatment further includes the step of: applying a hair treating liquid containing gold nanoparticles to the hair. Also disclosed is a hair treating liquid for hair shaping treatment that is applied to hair in hair shaping treatment, the hair treating liquid including gold nanoparticles.

## Description

### [Technical Field]

The present invention relates to a hair treating liquid for alleviating the damage sustained by hair during treatment in hair shaping treatment such as hair straightening treatment and permanent waving treatment, and a method for hair shaping treatment using the same.

### [Background Art]

Conventionally, methods for hair shaping treatment using a perm liquid made of a combination of a first agent containing a reducing agent and a second agent containing an oxidizing agent are widely known. Specifically, hair straightening treatment, which is a cosmetic technique for straightening curly or frizzy hair into straight hair, and permanent waving treatment that provides a hair with a configuration such as curls or waves. Generally, the following basic principle is used for the hair shaping treatment method. First, a first agent containing a reducing agent such as thioglycolic acid is applied to hair, thereby cleaving the disulfide bonds (cystine bonds) cross-linking the keratin constituting the hair. By eliminating the cross-linked structure of the keratin by cleaving the disulfide bonds, the binding by the cross-link of the keratin is eliminated. After shaping the hair in a state in which the cross-linked structure of the keratin has been eliminated, a second agent containing an oxidizing agent such as a hydrogen peroxide solution or sodium bromate is applied to the hair, thereby reforming the disulfide bonds. By reforming the disulfide bonds in the shaped state to cross-link the keratin, the configuration of the shaped hair is fixed. In the case of hair straightening treatment, the hair in which the disulfide bonds have been cleaved is subjected to iron pressing, to shape the hair into straight hair, followed by reforming the disulfide bonds. After shaping the hair into straight hair, the disulfide bonds are reformed, thereby fixing the straight configuration of the hair. In the case of permanent waving treatment, the first agent is applied to hair, and the hair is left for a predetermined period of time, thereby cleaving the disulfide bonds. Then, after washing off the first agent, the second agent is applied to the hair, with the hair being wound around cylindrical rods for providing a configuration such as waves, thereby reforming the disulfide bonds to fix the permanent waves of the hair.

Such a hair shaping treatment method using a perm liquid may impose a significant burden on hair, contrary to the expectations before treatment. Specifically, split hair, which is hair that has been split due to a reduced strength, or brittle hair, which is tightly curled hair with fine waves, may be generated. In particular, brittle hair or split hair tends to be generated at portions that are susceptible to a damage, such as a forelock and a hair at the nape of the neck. The cause of the generation of such brittle hair or split hair is considered to be that hair that has sustained a damage by hair dyeing or the past hair shaping is further subjected to a significant damage, for example, by an excessively long leaving time after application of the first agent or the selection of mismatching agents.

To perform treatment, a practitioner takes time to counsel a person to be treated, to have knowledge of the hair quality, the strength of waviness, the history of the treatments received by the hair, and the like. Then, the practitioner performs selection of agents, the determination of locations where the agents are to be applied, the selection of the leaving time, and the like. In addition, the practitioner performs treatment with utmost caution, while checking the state, such as the softening degree, of the hair to which the first agent has been applied. Despite of this, there have been increasing cases where brittle hair or split hair is generated contrary to the expectations.

As a method for hair straightening treatment that can achieve appropriate hair straightening, suppression of deterioration in the touch of hair or suppression of damage to hair, PTL 1 below discloses a method for hair straightening treatment that used a first agent in which a reducing agent and an alkaline agent are blended, and a second agent in which an oxidizing agent is blended. The method includes applying a hair straightening treatment auxiliary agent containing a hydrogen solution and having a hydrogen concentration of 1 mass% or more to hair, at least either before applying the first agent to the hair, while leaving the hair after the first agent has been applied to the hair, or before applying the second agent to the hair. Further, PTL 1 discloses that when the hair straightening auxiliary agent contains a noble metal colloid such as a platinum colloid or a palladium colloid, the noble metal colloid functions as a catalyst for promoting the reaction between a high-concentration hydrogen solution and hydroxy radicals, or as a catalyst for decomposing hydrogen peroxide serving as the oxidizing agent.

### [Citation List]

### [Patent Literature]

[PTL 1] Japanese Laid-Open Patent Publication No. 2015-17064

### [Summary of Invention]

### [Technical Problem]

It is an object of the present invention to provide a hair treating liquid and a method for hair shaping treatment for suppressing a damage caused by treatment in hair shaping treatment such as hair straightening treatment or permanent waving treatment.

### [Solution to Problem]

An aspect of the present invention is directed to a method for hair shaping treatment, including at least the steps of: applying a first agent containing a reducing agent to hair, and leaving the hair for a predetermined period of time; washing off the first agent applied to the hair; applying a second agent containing an oxidizing agent to the hair, and leaving the hair for a predetermined period of time; and washing off the second agent, wherein the hair shaping treatment method further includes the step of: applying a hair treating liquid containing gold nanoparticles to the hair. With such a hair shaping treatment method, it is possible to significantly reduce the frequency of generation of brittle hair or split hair. Furthermore, the hair to which the hair treating liquid containing gold nanoparticles has been applied exhibits excellent shaping properties and combability.

In hair shaping treatment such as hair straightening treatment or permanent waving treatment, the disulfide bonds (-S-S-) of the keratin are reduced to thiol groups (-SH) by the reducing agent contained in the first agent. The reduction of the disulfide bonds eliminates the cross-linked structure of the keratin. Then, the hair is shaped in a state in which the cross-linked structure of the keratin has been eliminated, and the shaped hair is treated with the second agent, thus oxidizing the thiol groups (-SH) of the keratin to reform the disulfide bonds (-S-S-). This reaction is a reversible redox reaction as represented by the following formula:

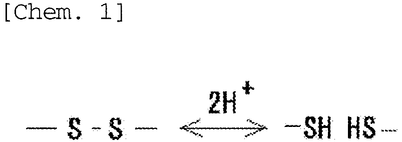

The present inventors have found that the above-described method can significantly reduce the frequency of generation of brittle hair or split hair. Then, the inventors presume that the mechanism is as follows. By applying the first agent to hair to reduce the disulfide bonds, thiol groups having higher nucleophilicity are produced. Because of their high nucleophilicity, thiol groups try to promptly transition to a stable oxidized state. If the reduction proceeds too rapidly at this time, the oxidation reaction also rapidly proceeds, so that the disulfide bonds may be reformed between thiol groups that are not supposed to react, or the thiol groups may react with an oxidizing substance contained in the hair or with the air, to produce an oxidation product that does not form a cross-linked structure. The inventors believe that this results in formation of a cross-linked structure that leaves a distortion in the keratin, or production of an oxidation product that has failed to form a cross-linked structure, thus generating brittle hair or split hair.

The generation of split hair or brittle hair tends to occur, for example, when the leaving time in the reduced state after application of the first agent is excessively longer than an appropriate time, or when a strong agent is selected. An appropriate leaving time after application of the first agent or an optimum agent varies depending on the amount of the damage sustained by the hair. For hair that has sustained a small amount of damage, the damage is unlikely to become manifest even when the hair is left for a relatively long period of time, or when a strongly alkaline agent is used. On the other hand, for hair that has sustained a large amount of damage, the damage is likely to become manifest in a short period of time. That is, it is believed that the hair that has sustained a large amount of damage tends to form a cross-linked structure that may leave a distortion, or tends to produce an oxidation product that has failed to form a cross-linked structure.

The present inventors believe that, in the case where gold nanoparticles are present when the first agent is applied in hair straightening treatment or permanent waving treatment, as shown in the following formula (2): 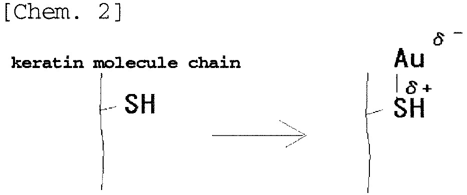 nucleophilicity is suppressed as a result of gold being ligated with the sulfur in the thiol groups (-SH) to stabilize the thiol groups (-SH). This has been derived based on the knowledge that the reduction rate of the thiol groups (-SH) is slowed when gold nanoparticles are present. Then, by slowly causing the reduction reaction to proceed, the rapid oxidation reaction by the nucleophilicity of the thiol groups (-SH) is also suppressed, as a result of which a cross-linked structure that leaves a distortion, or undesirable oxidation product becomes unlikely to be formed. As a result, the inventors found that the frequency of generation of brittle hair or split hair can be significantly reduced when hair straightening treatment or permanent waving treatment is performed by applying the first agent in the presence of gold nanoparticles.

It is preferable that the gold nanoparticles in the hair treating liquid have a concentration of 0.0001 mass% or more, from the viewpoint that the generation of brittle hair and the generation of split hair can be further suppressed by sufficiently controlling any undesirable reaction. Furthermore, it is preferable that the hair treating liquid further contains platinum nanoparticles, from the viewpoint that the platinum nanoparticles function as a catalyst for decomposing hydrogen peroxide serving as the oxidizing agent.

Furthermore, it is preferable that the step of applying the hair treating liquid to the hair is provided prior to application of the second agent to the hair, from the viewpoint that it is possible to inhibit the reduction reaction of the disulfide bonds of keratin from advancing too rapidly.

Furthermore, it is preferable that the first agent has a pH of 5 to 8, from the viewpoint that it is possible to inhibit cleavage of the ionic bonds of keratin that may cause the generation of brittle hair or split hair.

Furthermore, it is preferable that the method for hair shaping treatment is a method for hair straightening treatment, and further includes the step of: straightening the hair from which the first agent has been washed off into straight hair, while heating the hair. With such a hair straightening treatment method, it is possible to significantly reduce the frequency of generation of brittle hair or split hair. Furthermore, as a secondary effect, the hair to which the hair treating liquid containing gold nanoparticles has been applied exhibits excellent combability, and thus has enhanced shaping properties.

Furthermore, it is preferable that the method for hair straightening treatment further includes the step of: drying the hair from which the first agent has been washed off, and combing the hair while applying by spraying the hair treating liquid to the hair. In hair straightening treatment, before drying the hair from which the first agent has been washed off, the step of combing the hair so as to shape the hair into a straight configuration is often provided. In such a combing step, the amount of damage sustained by hair may be increased. Hair in a good condition is less likely to be tangled during combing, and has good combability. On the other hand, hair in a poor condition in which the hair has already contained brittle hair or split hair and a large amount of split hair before being treated may be more likely to be tangled during combing and have very poor combability. In such a case, when the speed at which the comb is moved is too fast, entanglement frequently occurs in the hair, so that the hair may sustain a damage. By applying gold nanocolloid to hair before combing, or during combing, the combability of the hair becomes significantly improved. As the result, it is possible to suppress a damage inflicted on the hair in combing.

Furthermore, it is preferable that the heating of the hair is performed with a hair iron set at 110 to 180°C. For heating using a hair iron in hair straightening treatment, a high temperature of about 180 to 200°C is often employed. Heating using a high-temperature hair iron causes a damage to the hair. The hair to which the hair treating liquid containing gold nanoparticles has been applied exhibits enhanced shaping properties, and therefore can be sufficiently shaped with a hair iron set at a low temperature of 110 to 180°C. The present inventors believe that the reason for this is that gold, which has a very high thermal conductivity, enhances the heat transfer efficiency of the hair iron.

Furthermore, it is preferable that the method for hair shaping treatment is a method for permanent waving treatment, and further includes the step of: shaping the hair by winding the hair around a rod. With such a permanent waving treatment method, it is possible to significantly reduce the frequency of generation of brittle hair or split hair. Furthermore, the hair maintains its resilience when the rod is removed from the hair wound around the rod. Accordingly, it is possible to achieve permanent waving treatment capable of properly retaining ridge-like waviness.

It is also preferable to further include, as with digital perming, the step of: heating the rod to 50°C or higher, with the hair being wound around the rod, from the viewpoint that the damage caused to the hair can be reduced.

Another aspect of the present invention is directed to a hair treating liquid for hair shaping treatment that is applied to hair in hair shaping treatment that is permanent waving treatment or hair straightening treatment, the hair treating liquid including gold nanoparticles. Such a hair treating liquid is preferable in that the damage sustained by hair can be reduced during or after hair shaping such as hair straightening treatment or permanent waving treatment.

### [Advantageous Effects of Invention]

According to the present invention, it is possible to reduce the damage inflicted on hair that may cause the generation of brittle hair or split hair in performing hair straightening treatment or permanent waving treatment.

### [Brief Description of Drawings]

[FIG. 1] FIG. 1 is a flowchart for illustrating a flow of hair straightening treatment according to a first embodiment.
[FIG. 2] FIG. 2 is a flowchart for illustrating a flow of permanent waving treatment according to a second embodiment.
[FIG. 3] FIG. 3 is an explanatory diagram for illustrating the retention of ridge-like waviness of hair when the hair is released from rods during permanent waving treatment, according to the second embodiment.

### [Description of Embodiments]

### [First Embodiment]

As a method for hair shaping treatment according to the present invention, an embodiment of a method for hair straightening treatment will be described with reference to the drawings.

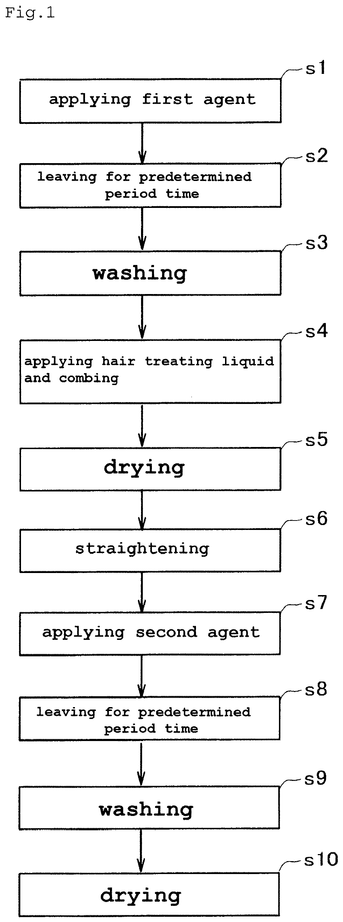

FIG. 1 is a flowchart for illustrating a flow of the steps of a method for hair straightening treatment according to the present embodiment.

In the hair straightening treatment method of the present embodiment, first, a first agent containing a reducing agent is applied to hair (s1), and the hair is left for a predetermined period of time (s2), thereby reducing the disulfide bonds cross-linking the keratin that forms the hair into thiol groups (-SH). In this step, the degree of freedom of the molecular chain of the keratin is increased by cleaving the cross-linked structure formed by the disulfide bonds that is formed in a distorted state so as to form curly or frizzy hair.

The first agent contains a reducing agent and other components that are optionally blended.

Specific examples of the reducing agent include thioglycolic acid, thioglycolates such as sodium thioglycolate and ammonium thioglycolate, thiolactic acid, cysteine, cysteamine, acetyl cysteine, thio glycerin, and sulfites.

Examples of the other components that are optionally blended include cream bases such as cetanol; an alkaline agent serving as a component that promotes penetration of the reducing agent by opening the cuticle by swelling the hair; nonionic surfactants that enhance the permeability of the reducing agent and function as emulsifiers for various components; cationic surfactants for imparting slickness to hair; oily components such as lanolin; NMF (natural moisturizing factor) and other moisturizers; and treatment components composed of protein, polypeptide, which is a hydrolysate thereof, amino acid, or the like. These may be used alone or in a combination of two or more.

Specific examples of the alkaline agent include ammonia water, ammonium carbonate, sodium carbonate, monoethanolamine, ammonium hydrogencarbonate, and arginine. Among these, ammonia water and ammonia hydrogen carbonate water are particularly preferable.

Note that in the hair straightening treatment method of the present embodiment, it is preferable that no alkaline agent is contained, or the amount of the alkaline agent is small from the viewpoint of reducing a damage inflicted on the hair. In the case of using a strongly alkaline first agent, there is a tendency that the ionic bonds of the keratin are cleaved, and brittle hair or split hair is more likely to be generated. Therefore, in the hair straightening treatment method of the present embodiment, the first agent has preferably a pH of 5 to 8, more preferably a pH of 5 to 7, particularly preferably a pH of 5 to 6.5.

The hair to which the first agent has been applied is optionally wrapped, and is subsequently left for a predetermined period of time (s2). At this time, optionally, it is preferable that the hair is shaped into straight hair by combing the hair. The leaving time is determined based on hair quality, the strength of waviness, the amount of damage that has been sustained by hair, and the like, while checking the change in the softening degree of the hair. In general, it is preferable that the hair is left preferably for about 5 to 30 minutes, more preferably for about 10 to 30 minutes, particularly preferably for about 15 to 20 minutes. When the hair has a strong waviness, optionally, the hair may be left, while heating hair within the range that does not inflict a damage on the hair.

Then, the first agent as described above is applied to the hair, and the hair is left for a predetermined period of time. Thereafter, washing with water, or optionally shampooing is performed to wash off the first agent (s3).

Then, preferably, while combing, or before combing the washed wet hair, a hair treating liquid containing gold nanoparticles as a hair straightening auxiliary agent is applied to a part or whole of the hair (s4). By stretching the washed wet hair by combing, the hair straightening treatment effect is enhanced. At this time, the generation of brittle hair or split hair can be suppressed by applying a hair treating liquid containing gold nanoparticles.

The hair to which the hair treating liquid containing gold nanoparticles has been applied exhibits improved combability. During combing, the state of damage of hair may become manifest to some degree. Hair in a good condition is less likely to be entangled during combing, and has good combability. On the other hand, hair in a bad condition tends to be entangled during combing, and often has poor combability. In the case of poor combability, entanglement frequently occurs in the hair when moving the comb fast, and the hair is caught in the comb, resulting in a damage to the hair. In such a case, the hair to which the hair treating liquid containing gold nanoparticles has been applied exhibits significantly improved combability. As a result, the generation of damage during combing that may be a cause of the generation of brittle hair or split hair can be suppressed.

The hair treating liquid containing gold nanoparticles is, for example, an aqueous treating liquid containing gold nanocolloid in which gold nanoparticles having a particle diameter of several tens of nm is dispersed in an aqueous medium. Although the particle diameter of the gold nanoparticles is not particularly limited as long as the gold nanoparticles can form a colloid, it is preferable that the colloid contains gold nanoparticles of preferably about 1 to 500 nm, more preferably about 5 to 300 nm, particularly preferably about 10 to 100 nm, especially preferably about 10 to 50 nm, in terms of ready availability.

The hair treating liquid to be applied contains gold nanoparticles having a high concentration of preferably 0.00001 mass% or more, more preferably 0.0001 mass% or more, particularly preferably 0.001 mass% or more. In the case of using the hair treating liquid containing gold nanoparticles having a high concentration, the effect of suppressing the generation of brittle hair or split hair or improving the combability can be significantly increased. Furthermore, the upper limit of the concentration of the gold nanoparticles in the hair treating liquid is not particularly limited, but is preferably 0.1 mass%, more preferably 0.01 mass%, in terms of the cost and the stability of the colloid.

Furthermore, the hair treating liquid may contain platinum nanocolloid together with gold nanocolloid. The platinum nanocolloid is preferable in that it functions as a catalyst for promoting decomposition of hydrogen peroxide in the case of using a hydrogen peroxide solution as the oxidizing agent.

Note that, in the present embodiment, an example has been described in which the hair treating liquid containing gold nanoparticles is applied to the wet hair after washing (s3). The hair treating liquid containing gold nanoparticles is preferably applied in a step prior to the step of applying the second agent, particularly preferably applied to the wet hair from which the first agent has been washed off, in that it is possible to suppress the reduction reaction from excessively rapidly proceeding before manifestation of brittle hair or split hair. On the other hand, the hair treating liquid containing gold nanoparticles may be applied in any step between a step prior to the application of the first agent and the end of treatment, or may be applied after treatment. Alternatively, the aforementioned hair treating liquid may be applied a plurality of times in portions, rather than at once. It seems that the redox reaction by hair straightening treatment is not completed immediately after the treatment, but proceeds for several days after the treatment. Therefore, the effect of suppressing an undesirable redox reaction can be achieved at any point of time before the treatment, during the treatment, or after the treatment.

The amount of the hair treating liquid containing gold nanoparticles to be applied to hair is dependent on the amount of hair, and thus cannot be definitely specified. However, it is preferable that the hair treating liquid is applied repeatedly little by little in a single treatment by spraying or the like. Specifically, it is preferable that the hair treating liquid is applied such that the gold nanoparticles are attached, for example, in an amount of preferably about 0.01 to 100 mg, more preferably about 1 to 10 mg, particularly preferably about 2 to 10 mg.

Then, drying is performed using a hair drier (s5). Although the drying condition is not particularly limited, it is preferable to dry the hair at a temperature of about 100 to 150°C. During drying, a portion that has sustained a large amount of damage may become manifest. In such a case, to effectively suppress the generation of brittle hair or split hair, the hair treating liquid containing gold nanoparticles may be applied locally to the portion whose damage has become manifest.

Then, the dried hair is straightened into straight hair by heating (s6). The step of straightening hair into a straight configuration by heating is a step of shaping, by iron pressing, the hair containing the keratin in which the cross-linked structure by the disulfide bonds has been eliminated with the first agent, into a straight configuration. Although the set temperature for iron pressing is not particularly limited, iron pressing is performed preferably at a temperature of about 100 to 200°C. A portion that has sustained a large amount of damage may become manifest also during straightening by heating. In such a case, to suppress the generation of brittle hair or split hair more effectively, the hair treating liquid containing gold nanoparticles may be applied locally to the portion whose damage has become manifest.

Note that in the hair straightening treatment of the present embodiment, it is preferable to perform iron pressing by using a hair iron at a set temperature of particularly preferably about 110 to 180°C, more preferably at a low temperature of about 150 to 170°C. In straightening by heating with a hair iron, a high temperature of about 180 to 200°C is widely used. However, the hair to which the hair treating liquid containing gold nanoparticles has been applied exhibits enhanced shaping properties, and thus can be sufficiently shaped with a hair iron at a low temperature. The present inventors believe that the reason is that gold has a higher thermal conductivity (318 W/(m/K) 300 K) than many other metals, and thus enhances the heat transfer efficiency of the hair iron.

Then, a second agent containing an oxidizing agent is applied to the hair that has been shaped into a straight configuration, and the hair is left for a predetermined period of time (s7).

As the second agent, any oxidizing agent that has been conventionally used for hair straightening treatment can be used without any particular limitation. Specific examples of such oxidizing agents include a hydrogen peroxide solution and bromate. The hydrogen peroxide solution is usually contained within the range of 2.5% or less and a pH of 2.5 to 4.5. Usually, the bromate is preferably contained within the range of 3.2% or more and a pH of 4.0 to 9.0.

Then, after applying the second agent to the hair, the hair is left for a predetermined period of time, thereby reforming the disulfide bonds in the hair that has been straightened into straight hair (s8). Then, after leaving the hair for a predetermined period of time, the hair is washed to wash off the second agent (s9). In washing, shampooing and hair treatment are performed optionally. Then, after washing, drying such as towel drying or air drying is performed (s10). Through the steps described above, the hair straightening treatment of the present embodiment in which the generation of brittle hair or split hair has been suppressed is performed.

### [Second Embodiment]

An embodiment of the method for permanent waving treatment will be described in detail as the method for hair shaping treatment according to the present invention. Conventionally, when performing permanent waving treatment, to perform treatment, a practitioner takes time to counsel a person to be treated, to have knowledge of the hair quality, the strength of waviness, the history of the damages that have been sustained by hair, and the like. If it is determined that there is a high possibility that a damage such as brittle hair or split hair occurs in the case of performing permanent waving treatment, the practitioner may have to decide not to perform the treatment. With the permanent waving treatment method of the present embodiment, it is possible to suppress the generation of a damage such as brittle hair or split hair also for hair that has sustained a large amount of damage. Therefore, the standard for such determination as to whether to decide not to perform treatment can be further relaxed. In addition, the permanent waving treatment method of the present embodiment can provide excellent shaping properties at the time of performing permanent waving treatment, and achieve ridge-like waviness of waves that is close to that expected prior to treatment. Moreover, a good touch after treatment can be achieved.

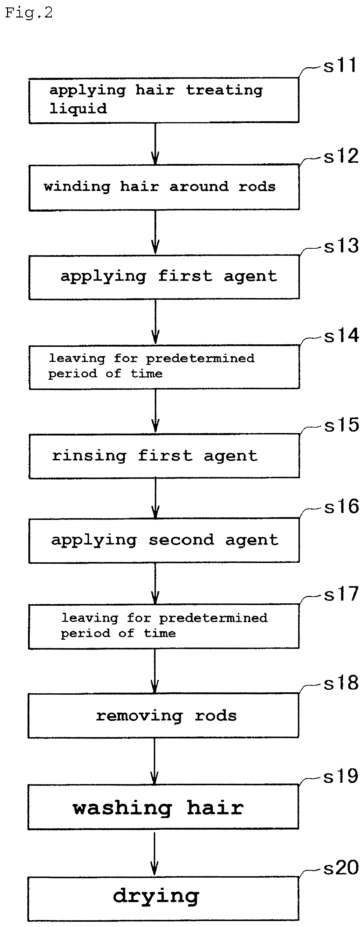

FIG. 2 is a flowchart for illustrating a flow of the steps of the method for permanent waving treatment according to the present embodiment.

In the permanent waving treatment method of the present embodiment, the same step of applying the hair treating liquid containing gold nanoparticles hair as the step described in the first embodiment is provided before the step of applying the first agent to the hair, prior to application of the second agent to the hair in permanent waving treatment.

In the permanent waving treatment method of the present embodiment, as an example, first, a hair treating liquid containing gold nanoparticles is applied to hair (s11). Then, the hair to which the hair treating liquid containing gold nanoparticles has been applied is wound around cylindrical rods having a diameter suitable for imparting a waving configuration (winding) (s12). Then, the first agent is applied to the hair that is wound around the rods (s13), and the hair is left for a predetermined period of time (s14). By applying the first agent to the hair and leaving the hair for a predetermined period of time, the disulfide bonds cross-linking the keratin are reduced into thiol groups. In this step, the degree of freedom is imparted to the molecular chain of the keratin by cleaving the cross-linked structure formed by the disulfide bonds. At this time, the hair treating liquid containing gold nanoparticles may be further applied to the hair to which the first agent has been applied.

As the first agent, the same first agent as that described with regard to the hair straightening treatment method described in the first embodiment can be used. In addition, it is preferable that the first agent contains no alkaline agent from the viewpoint of suppressing the generation of brittle hair or split hair. When the first agent is made alkaline by inclusion of an alkaline agent therein, the ionic bonds of the keratin are cleaved to reduce the strength of the hair, so that brittle hair or split hair tends to be generated. Therefore, in the permanent waving treatment method of the present embodiment as well, the first agent has preferably a pH of 5 to 8, more preferably a pH of 5 to 7, particularly preferably a pH of 5 to 6.5.

The hair to which the first agent and the hair treating liquid containing gold nanoparticles have been applied and that is wound around the rods are optionally wrapped, and is thereafter left for a predetermined period of time (s14). The leaving time is determined based on the hair quality, the strength of the reducing power of the first agent, the amount of damage that has been sustained by hair, and the like, while checking the change in the softening degree of the hair. In general, the hair is left preferably for about 5 to 30 minutes, more preferably for about 10 to 30 minutes, particularly preferably for about 15 to 20 minutes.

Then, after applying the first agent and the above-described hair treating liquid containing gold nanoparticles to the hair, and leaving the hair for a predetermined period of time, washing with water or shampooing is optionally performed, thereby rinsing the first agent (s15).

Then, the second agent containing an oxidizing agent is applied to the hair wound around the rods (s16), and the hair is left for a predetermined period of time (s17).

As the second agent, the same second agent as that described with regard to the hair straightening treatment method described in the first embodiment can also be used.

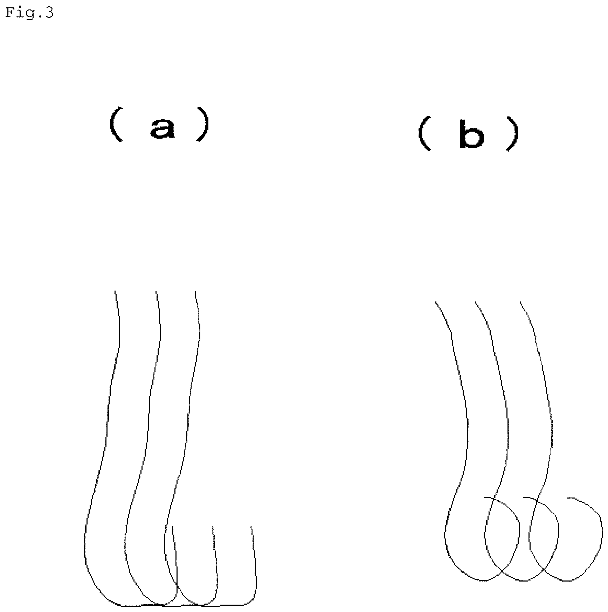

After applying the second agent to the hair, the hair is left for a predetermined period of time, thereby reforming the disulfide bonds in the hair wound the rods (s17). Then, after leaving the hair for a predetermined period of time, the rods are removed from the hair wound around the rods (s18). The effect achieved by applying the hair treating liquid containing gold nanoparticles in the permanent waving treatment method of the present embodiment is also observed in a significant manner for the retainability of the ridge-like waviness of waves at the time of removing the rods. With regard to the retainability of the ridge-like waviness at the time of removing the rods from the hair wound around the rods, the phenomenon "slackening" in which curls are more loosened by the gravity tends to occur when the hair treating liquid containing gold nanoparticles is not applied, as shown in FIG. 3(a). It has been confirmed that, by applying the hair treating liquid containing gold nanoparticles in the permanent waving treatment method of the present embodiment, the hair can maintain resilience and becomes "less likely to undergo slackening", thus reliably retaining the ridge-like waviness, as shown in FIG. 3(b).

Then, the hair is washed (s19). In washing, shampooing and hair treatment is optionally performed. Then, after washing, drying such as towel drying and air drying is performed (s20). Through these steps, the permanent waving treatment of the present embodiment that suppresses the generation of brittle hair or split hair is performed.

In the present embodiment, by applying the hair treating liquid containing gold nanoparticles before, during, or immediately after applying the first agent, the reaction by the first agent can be made gradual so as to suppress a rapid cleavage of the disulfide bonds. Accordingly, the present method is preferable in that it is possible to inhibit an undesirable reaction from taking place quickly, facilitating control of a damage inflicted on the hair. Note that the hair treating liquid containing gold nanoparticles may be applied in any step until the end of treatment, or after treatment. Alternatively, the aforementioned hair treating liquid may be applied a plurality of times in portions, rather than at once. The redox reaction in permanent waving treatment is not completed immediately after the treatment, but proceeds for several days after the treatment. Therefore, the effect of suppressing an undesirable redox reaction can be achieved at any point of time before the treatment, during the treatment, or after the treatment.

### [Third Embodiment]

In the second embodiment, general permanent waving treatment is described as a typical example of permanent waving treatment. When stronger shaping that is required to provide strong curls or shape-memory curls is necessary, it is possible to use hot permanent called "digital perming," which includes the step of heating with heaters included in rods in a state in which hair is wound around the rods. In the following, a method for permanent waving treatment that uses digital perming will be described.

In the permanent waving treatment method of the present embodiment, first, a hair treating liquid containing gold nanoparticles is evenly applied by spraying to hair. Then, a first agent containing a reducing agent is applied to the hair to which the hair treating liquid has been applied, and the hair is left for a predetermined period of time, thereby reducing the disulfide bonds to eliminate the cross-linked structure. At this time, the leaving time is such that the hair is left for preferably about 5 to 20 minutes, more preferably about 10 to 15 minutes, so as to have a moderate softness, while monitoring the softening degree of the hair. Then, after leaving the hair for a predetermined period of time, the first agent is washed off. Note that it is preferable to also apply by spraying the hair treating liquid containing gold nanoparticles to the wet hair from which the first agent has been washed off.

Then, the wet hair from which the first agent has been washed off, and to which the hair treating liquid containing gold nanoparticles has been evenly applied is wound around a plurality of rods each including a heater. Then, the electrodes of the rods are connected to a controller, and the rods are heated for a predetermined period of time under the temperature control by the controller. The set temperature for heating is preferably 40 to 60°C, more preferably 45 to 55°C. In addition, the heating time is preferably about 5 to 25 minutes, more preferably about 10 to 20 minutes. Then, after heating, the rods are removed from the hair. Then, the hair treating liquid containing gold nanoparticles and optionally other treatment agents are applied to the hair. Then, the second agent is applied to the hair, and the hair is left for a predetermined period of time, thereby oxidizing the thiol groups to form a cross-linked structure. The leaving time is preferably about 0 to 10 minutes, more preferably about 5 to 10 minutes. Then, after leaving the hair, the second agent is washed off. It is preferable to further apply the hair treating liquid containing gold nanoparticles to the hair after washing off the second agent. Then, as finishing, the hair is conditioned by being blow-dried.

It has also been confirmed that, in the case of using hot permanent such as digital perming as the permanent waving treatment method of the present embodiment, the generation of brittle hair or split hair can be suppressed, and the hair can maintain resilience and becomes "less likely to undergo slackening", thus reliably retaining the ridge-like waviness.

### [Examples]

Hereinafter, the present invention will be described in further detail by way of examples. It should be appreciated that the scope of the present invention is by no means limited by the examples.

### (Example 1)

To the hair of a female subject that had sustained a somewhat large amount of damage, and had slightly conspicuous split hair and curly or frizzy hair, a first agent was applied, while combing the hair. Note that the first agent was prepared with a formulation in which 6 mass% of ammonium thioglycolate was contained in a cream base containing cetanol as a main component so as to have a pH of 4.5.

Then, the hair was left as it was for about 20 minutes, while checking the softening degree of the hair. Then, the hair was shampooed. Then, undiluted gold nanocolloid (concentration of gold contained: 0.005 mass%) was evenly sprayed to the entirety of the wet hair after being shampooed. Then, the hair was straightened by being combed with a comb. The combability at this time was evaluated according to the following criteria.

### (Combability)

A: Excellent combability: the comb was hardly caught in entangled hair.
B: Good combability, slightly inferior to A: the comb was slightly caught in entangled hair.
C: Poor combability: the comb was frequently caught in entangled hair, and combing was not possible unless the comb was moved slowly.

As the result of evaluation, the combability was "A".

Then, the combed hair was dried with a hair drier. Then, the dried hair was shaped into a straight configuration by being stretched with a hair iron set at 160°C. Then, a second agent was applied to the hair that had been shaped into a straight configuration, and the hair was left as it was for about 10 minutes. Thereafter, the hair was washed with water, and further dried with a hair drier. Note that as the second agent, a second agent prepared with a formulation in which 6 mass% of a hydrogen peroxide solution was adjusted with water to about 1.5 mass% was used.

In this manner, hair straightening was performed. Then, the resulting hair was evaluated according to the following criteria.

### (Generation of split hair or brittle hair)

A: Substantially no split hair or brittle hair was recognized by careful observation.
B: Split hair or brittle hair was generated sufficient to be recognized by careful observation.
C: Split hair and brittle hair were generated sufficient to be significantly recognized from the appearance.

The resulting hair did not undergo generation of brittle hair or split hair, and was straightened into natural-like straight hair. In addition, no brittle hair was generated after an elapse of one week after performing the treatment. The results are shown in Table 1 below.

**[Table 1]**

| Example No. | Damage | Leaving time (min) | Hair treating liquid | Combability | Generation of brittle hair or split hair | After one week |
|---|---|---|---|---|---|---|
| 1 | Somewhat large | 20 | Gold colloid (0.005 mass%) | A | A | None |
| 2 | Large | 30 | Gold colloid (0.005 mass%) | A | A | None |
| 3 | Significantly large | 30 | Gold colloid (0.005 mass%) | A | A | None |
| 4 | Large | 30 | Gold colloid (0.0005 mass%) | A | A | None |
| 5 | Large | 30 | Gold colloid (0.00005 mass%) | B | A | Tendency present |
| Com. Ex. 1 | Somewhat large | 20 | None | C | C | - |
| Com. Ex. 2 | Large | 30 | Platinum colloid (0.005 mass%) | C | C | - |

### (Example 2)

On the hair of another female subject that had sustained a larger amount of damage than that in Example 1, and had conspicuous split hair and curly or frizzy hair, hair straightening was performed in the same manner as in Example 1 except that the leaving time after application of the first agent was changed to about 30 minutes. Note that during combing, the combability was excellent as with Example 1, and the combability was evaluated as "A". The resulting hair did not undergo generation of brittle hair or split hair, and was straightened into natural-like straight hair. In addition, no brittle hair was generated after an elapse of one week after performing the treatment.

### (Example 3)

On another female subject who had hair that had sustained a larger amount of damage than the subject of Example 2, and also had conspicuous split hair, hair straightening treatment was performed in the same manner as in Example 2. Note that during combing, the combability was excellent as with Example 2, and the combability was evaluated as "A". The resulting hair did not undergo generation of brittle hair or split hair, and was straightened into natural-like straight hair. In addition, no brittle hair was generated after an elapse of one week after performing the treatment.

### (Example 4)

On another female subject who had hair that had sustained the same level of damage as the female subject of Example 2, the hair straightening treatment was performed in the same manner as in Example 2 except that a 10-fold dilution of undiluted gold nanocolloid was applied, instead of applying undiluted gold nanocolloid. As with Example 2, the combability was excellent, and the combability was evaluated as "A". In addition, the resulting hair did not undergo generation of brittle hair or split hair, and was straightened into natural-like straight hair. In addition, no brittle hair was generated after an elapse of one week after performing the treatment.

### (Example 5)

On another female subject who had hair that had sustained the same level of damage as the female subject of Example 2, the hair straightening treatment was performed in the same manner as in Example 2 except that a 100-fold dilution of undiluted gold nanocolloid was applied, instead of applying undiluted gold nanocolloid. Note that during combing, the combability was slightly inferior to that in Example 4, but was favorable. The comb was slightly caught in entangled hair, and the combability was evaluated as "B". The resulting hair did not undergo generation of brittle hair or split hair, and was straightened into natural-like straight hair. However, a slight tendency of generation of brittle hair was observed after an elapse of one week after the treatment.

### (Comparative Example 1)

On another female subject who had hair that had sustained the same level of damage as the female subject of Example 1, the hair straightening treatment was performed in the same manner as in Example 1 except for omitting the step of applying gold nanocolloid. At this time, the combability during combing was poor, and the combability was evaluated as "C". In addition, brittle hair was generated in the nape portion of the neck of the resulting hair. Further, the hair was straightened into straight hair in which slightly curly or frizzy hair slightly remained.

### (Comparative Example 2)

On another female subject who had hair that had sustained the same level of damage as the female subject of Example 2, the hair straightening treatment was performed in the same manner as in Example 2 except that undiluted platinum nanocolloid (concentration of platinum contained: 0.005 mass%) was applied, instead of applying gold nanocolloid. At this time, the combability during combing was poor, and the combability was evaluated as "C". Brittle hair was generated in the nape portion of the neck of the resulting hair.

In the foregoing examples, after applying the first agent, the hair was left for 20 to 30 minutes. The reason is that for the evaluation purpose, the leaving time was set to be slightly longer than that in a usual hair straightening treatment.

The results of the examples demonstrate the following. For the female subjects of Examples 1 to 3 in which undiluted gold nanocolloid was sprayed, and the female subject of Example 4 in which a 10-fold dilution of undiluted gold nanocolloid was sprayed, the hair was successfully finished into natural-like straight hair without generation of brittle hair or split hair, regardless of the amount of damage and the leaving time. In addition, the combability during combing was good. On the other hand, when the concentration of the gold nanocolloid was low, brittle hair tended to be generated after the treatment, as shown in Example 5.

On the other hand, for the female subject of Comparative Example 1 in which the amount of damage and the leaving time were the same levels as those in the Example 1, and gold nanocolloid was not sprayed, brittle hair was generated at the nape of the neck. In addition, the combability during combing was poor. On the other hand, for the female subject of Comparative Example 2 in which the amount of damage and the leaving time were the same levels as those in the Example 2, and platinum nanocolloid was sprayed in place of gold nanocolloid, brittle hair was also generated at the nape of the neck. Further, no improvement was observed in the combability during combing.

### (Example 6)

To the entirety of the hair of a female subject who had the history of hair straightening treatment, had hair that had sustained a somewhat large amount of damage, and slightly conspicuous split hair, undiluted gold nanocolloid (concentration of gold contained: 0.005 mass%) was evenly sprayed. Then, the hair to which the gold nanocolloid had been applied was wound around rods. After applying a first agent to the hair, undiluted gold nanocolloid was further sprayed, and thereafter, the hair was left for 15 minutes. Note that the first agent was prepared with a formulation in which 6 mass% of ammonium thioglycolate was contained in a cream base containing cetanol as a main component so as to have a pH of 4.5.

Then, the hair wound around the rods were rinsed with warm water, thereby washing off the first agent. Then, a second agent was applied to the rinsed wet hair, and the hair was left as it was for about 10 minutes, while checking the curling state in the middle of the leaving. Note that the second agent used here was prepared with a formulation in which 6 mass% of a hydrogen peroxide liquid was adjusted to about 1.5 mass% with water. Then, the rods were removed from the hair. Then, the hair was shampooed, thereby removing the second agent. Then, the hair was blow-dried with a hair drier. Then, the generation of split hair or brittle hair was evaluated according to the same criteria as those in Example 1. In addition, the shaping quality and the finished quality perceived by the practitioner immediately after removing the rods were evaluated according to the following criteria.

### (Shaping quality perceived by practitioner immediately after removing rods)

A: The hair was shaped to have strong resilience and firm curls.
B: The hair had no resilience, and the hair was sagged by the gravity and had loose curls.

### (Finished quality)

A: Firm curls that had been intended to be provided with the rod diameter, and had elasticity and a high level of ridge-like waviness were obtained.
B: Curls that had been intended to be obtained with the rod diameter were not obtained, and loose curls having a low level of ridge-like waviness were obtained.

According to the evaluation performed by the practitioner immediately after removing the rods, the hair was shaped to have strong resilience and firm curls. In addition, there was no generation of brittle hair or split hair in the finished hair, and permanent waves with high ridge-like waviness were shaped. The results are collectively shown in Table 2 below.

**[Table 2]**

| Example No. | Damage | Hair treating liquid | Shaping quality perceived by practitioner immediately after removing rods | Generation of brittle hair or split hair | Finished quality |
|---|---|---|---|---|---|
| 6 | Large | Gold colloid (0.005 mass%) | A | A | A |
| 7 | Large | Gold colloid (0.0005 mass%) | A | A | A |
| 8 | Large | Gold colloid (0.00005 mass%) | B | A | A |
| Com. Ex. 3 | Large | None | B | C | B |
| Com. Ex. 4 | Large | Platinum colloid (0.005 mass%) | B | C | B |

### (Example 7)

On another female subject who had hair that had sustained the same level of damage as the female subject of Example 6, the permanent waving treatment was performed in the same manner as in Example 6 except that a 10-fold dilution of undiluted gold nanocolloid was applied, instead of applying undiluted gold nanocolloid. The results are shown in Table 2.

### (Example 8)

On another female subject who had hair that had sustained the same level of damage as the female subject of Example 6, the permanent waving treatment was performed in the same manner as in Example 6 except that a 100-fold dilution of undiluted gold nanocolloid was applied, instead of applying undiluted gold nanocolloid. The results are shown in Table 2.

### (Comparative Example 3)

On another female subject who had hair that had sustained the same level of damage as the female subject of Example 6, the permanent waving treatment was performed in the same manner as in Example 6 except for omitting the step of applying gold nanocolloid. The results are shown in Table 2.

### (Comparative Example 4)

On another female subject who had hair that had sustained the same level of damage as the female subject of Example 6, the permanent waving treatment was performed in the same manner as in Example 6 except that undiluted platinum nanocolloid (concentration of platinum contained: 0.005 mass%) was applied, instead of applying undiluted gold nanocolloid. The results are shown in Table 2.

The results of Examples 6 to 8 and Comparative Examples 3 and 4 demonstrate the following. For the female subject of Example 6 in which undiluted gold nanocolloid was sprayed and the female subject of Example 7 in which a 10-fold dilution of undiluted gold nanocolloid was sprayed, no generation of split hair or brittle hair was observed, and curls were firmly shaped. In addition, the practitioner determined that the hair had strong resilience immediately after removing the rods, and the curls were not sagged by the gravity. On the other hand, when the concentration of the gold nanocolloid was low, the effect was slightly insufficient, as shown in Example 8.

Meanwhile, for the female subject of Comparative Example 3 in which gold nanocolloid was not sprayed and the female subject of Comparative Example 4 in which platinum nanocolloid was sprayed in place of gold nanocolloid, split hair or brittle hair was generated, and loose curls that were unlikely to exhibit ridge-like waviness were shaped. In addition, the practitioner determined that the hair did not have resilience immediately after removing the rods, and the curls were sagged by the gravity.

### (Example 9)

To the entirety of the hair of a female subject who had the hair that had sustained a somewhat large amount of damage, and slightly conspicuous split hair, a hair treating liquid that was undiluted gold nanocolloid (concentration of gold contained: 0.005 mass%) was evenly sprayed. Then, a first agent was applied to the hair to which the hair treating liquid had been applied. After further spraying the hair treating liquid to the hair, the hair was left for 15 minutes. Note that the first agent was prepared with a formulation in which 6 mass% of ammonium thioglycolate was contained in a cream base containing cetanol as a main component so as to have a pH of 4.5. Then, the hair was washed with warm water, thereby washing off the first agent. Then, the hair treating liquid was further sprayed to the hair.

Then, a second agent was applied to the wet hair from which the first agent had been washed off and to which the hair treating liquid had been evenly applied. Note that the second agent used was prepared with a formulation in which 6 mass% of a hydrogen peroxide solution had been adjusted to about 1.5 mass% with water. Then, the hair to which the second agent had been applied was wound around a plurality of rods each including a heater. Then, the electrodes of the rods were connected to a controller set at 45°C, and the rods were heated for 20 minutes. Then, after heating, the rods were removed from the hair, and the second agent was washed off. Then, the hair was blow-dried with a hair drier. Then, the shaping quality and the finished quality perceived by the practitioner immediately after removing the rods were evaluated according to the same criteria as those of Example 6

### (Shaping quality perceived by practitioner immediately after removing rods)

A: The hair was shaped to have strong resilience and firm curls.
B: The hair had no resilience, and the hair was sagged by the gravity and had loose curls.

### (Finished quality)

A: Firm curls that had been intended to be obtained with the rod diameter, and had elasticity and a high level of ridge-like waviness were obtained.
B: Curls that had been intended to be obtained with the rod diameter were not obtained, and loose curls having a low level of ridge-like waviness were obtained.

According to the evaluation made by the practitioner immediately after removing the rods, the hair was shaped to have strong resilience and firm curls. In addition, there was no generation of brittle hair or split hair in the finished hair, and permanent waves with high ridge-like waviness were shaped. The results are collectively shown in Table 3 below.

**[Table 3]**

| Example No. | Damage | Hair treating liquid | Shaping quality perceived by practitioner immediately after removing rods | Generation of brittle hair or split hair | Finished quality |
|---|---|---|---|---|---|
| 9 | Large | Gold colloid (0.005 mass%) | A | A | A |
| 10 | Large | Gold colloid (0.0005 mass%) | A | A | A |
| 11 | Large | Gold colloid (0.00005 mass%) | B | A | A |
| Com. Ex. 5 | Large | None | B | C | B |
| Com. Ex. 6 | Large | Platinum colloid (0.005 mass%) | B | C | B |

### (Example 10)

On another female subject who had hair that had sustained the same level of damage as the female subject of Example 9, the permanent waving treatment was performed in the same manner as in Example 6 except that a 10-fold dilution of undiluted gold nanocolloid was applied, instead of applying undiluted gold nanocolloid. The results are shown in Table 3.

### (Example 11)

On another female subject who had hair that had sustained the same level of damage as the female subject of Example 9, the permanent waving treatment was performed in the same manner as in Example 6 except that a 100-fold dilution of undiluted gold nanocolloid was applied, instead of applying undiluted gold nanocolloid. The results are shown in Table 3.

### (Comparative Example 5)

On another female subject who had hair that had sustained the same level of damage as the female subject of Example 9, the permanent waving treatment was performed in the same manner as in Example 6 except for omitting the step of applying gold nanocolloid. The results are shown in Table 3.

### (Comparative Example 6)

On another female subject who had hair that had sustained the same level of damage as the female subject of Example 9, the permanent waving treatment was performed in the same manner as in Example 6 except that undiluted platinum nanocolloid (concentration of platinum contained: 0.005 mass%) was applied, instead of applying undiluted gold nanocolloid. The results are shown in Table 3.

### [Industrial Applicability]

According to the present invention, it is possible to significantly reduce the manifestation of damages generated during treatment in hair treatment such as hair straightening treatment and permanent waving treatment.

## Claims

1. A method for hair shaping treatment, comprising at least the steps of:
applying a first agent containing a reducing agent to hair, and leaving the hair for a predetermined period of time;
washing off the first agent applied to the hair;
applying a second agent containing an oxidizing agent to the hair, and leaving the hair for a predetermined period of time; and
washing off the second agent, wherein
the hair shaping treatment method further comprises the step of:
applying a hair treating liquid containing gold nanoparticles to the hair.

2. The method for hair shaping treatment according to claim 1, wherein
the gold nanoparticles in the hair treating liquid have a concentration of 0.0001 mass% or more.

3. The method for hair shaping treatment according to claim 1 or 2, wherein
the hair treating liquid further contains platinum nanoparticles.

4. The method for hair shaping treatment according to any one of claims 1 to 3, wherein
the step of applying the hair treating liquid to the hair is provided prior to application of the second agent.

5. The method for hair shaping treatment according to any one of claims 1 to 4, wherein
the first agent has a pH of 5 to 8.

6. The method for hair shaping treatment according to any one of claims 1 to 5, wherein
the method for hair shaping treatment is a method for hair straightening treatment, and
further comprises the step of:
straightening the hair from which the first agent has been washed off into straight hair, while heating the hair.

7. The method for hair shaping treatment according to claim 6, further comprising the step of:
drying the hair from which the first agent has been washed off, and combing the hair while applying by spraying the hair treating liquid to the hair.

8. The method for hair shaping treatment according to claim 6 or 7, wherein
the heating is performed with a hair iron set at 110 to 180°C.

9. The method for hair shaping treatment according to any one of claims 6 to 8, wherein
the method for hair shaping treatment is a method for permanent waving treatment, and
further comprises the step of: shaping the hair by winding the hair around rods.

10. The method for hair shaping treatment according to claim 9, further comprising the step of:
heating the rods to 50°C or higher, with the hair being wound around the rods.

11. A hair treating liquid for hair shaping treatment that is applied to hair in hair shaping treatment that is hair straightening treatment or permanent waving treatment,
the hair treating liquid comprising gold nanoparticles.

12. The hair treating liquid for hair shaping treatment according to claim 11, wherein
the hair treating liquid comprises 0.0001 mass% or more of gold nanoparticles.

13. The hair treating liquid for hair shaping treatment according to claim 11 or 12,
further comprising platinum nanoparticles.
